# EUROPEAN PATENT APPLICATION

(11) **EP 3 854 757 A1**
(43) Date of publication of application: **28.07.2021**
(21) Application number: 20153457.5
(22) Date of filing: 23.01.2020
(51) Int. Cl.: C01G 47/00, A61K 51/12

(54) **RHENIUM SULFIDE PARTICLES AND MICROWAVE ASSISTED PROCESS FOR THEIR PREPARATION**

(71) Applicant: Technische Universität München, 80333 München (DE)
(72) Inventor: HEINZ, Werner, 86343 Königsbrunn (DE)
(74) Representative: Vossius & Partner Patentanwälte Rechtsanwälte mbB

(57) **Abstract**

Provided is a process for the preparation of particles comprising rhenium (VII) sulfide, which process comprises providing a reaction solution comprising perrhenic acid or a salt thereof, a Bronsted acid other than perrhenic acid and a sulfide source, and heating the reaction solution comprising perrhenic acid or a salt thereof, a Bronsted acid other than perrhenic acid, and a sulfide source by exposure to microwave radiation to convert the perrhenic acid or the salt thereof to particles comprising rhenium (VII) sulfide. As a further embodiment, composition are provided which comprise a plurality of particles comprising rhenium (VII) sulfide, said particles having a volume average particle size, of 0.05 to 5 µm, a unimodal particle size distribution and a polydispersity Ð in the range of 1.0 to 2.5.

## Description

The present invention provides rhenium sulfide particles, processes for their preparation and compositions containing them.

Worldwide, more than 5 million people develop skin cancer every year. 80% of these cases are non-melanoma skin cancer, divided into basal cell carcinomas and spinal cell carcinomas. Since 1994, the prevalence has increased by more than 300%. Non-melanoma skin cancer can be treated either surgically, with chemotherapy or non-invasive (radiotherapy, cryotherapy, laser therapy etc.). A non-invasive treatment is the local, superficial radioisotope therapy. Although surgical removal is considered to be the safest method of treatment, surgical treatment has certain post-operative disadvantages of scarring, pain, or possible infections, and can only be relied on if the affected area is not too large and the physical condition of the patient allows a surgical intervention. Due to severe side effects, chemotherapy is typically not used in an early stage, or where the cancer has not spread.

As a method for local treatment of skin cancer, epidermal radioisotope therapy involves the application of a radioisotope, to affected skin areas. The beta emitters rhenium-186 or rhenium-188 are suitable for this purpose. Advantages of epidermal radioisotope therapy are that it allows a pain free therapy, and frequently only one application of the radioisotopes is necessary. In addition, the therapy hardly causes any scars or damages in the surrounding tissue, so that it is particularly suitable for treatments of visible parts of the body, such as the face. In addition, this therapy can be used as a post-treatment after surgery to kill remaining tumor cells.

WO 2018/069329 discloses particles containing activated rhenium for use in radioisotope therapy and processes for their production, which involve the precipitation of rhenium compounds, such as rhenium (VII) sulfide. Still, a need remains for an efficient process which allows rhenium-containing particles to be prepared in a time efficient manner at a high yield, while allowing the particle size and the particle size distribution of the obtained particles to be controlled within desired limits.

To that extent, the present invention provides, as a first aspect, a process for the preparation of particles comprising rhenium (VII) sulfide, which process comprises
providing a reaction solution comprising perrhenic acid or a salt thereof, a Bronsted acid other than perrhenic acid, a sulfide source and optionally a capping agent, and
heating the reaction solution comprising perrhenic acid or a salt thereof, a Bronsted acid other than perrhenic acid, a sulfide source and optionally a capping agent by exposure to microwave radiation to convert the perrhenic acid or the salt thereof to particles comprising rhenium (VII) sulfide.

The process in accordance with the invention allows the rapid production of the particles comprising rhenium (VII) sulfide at excellent yields in terms of the rhenium or the rhenium compound used as a starting material. Since no alkali metal compounds and/or halogen compounds are needed, undesirable impurities of such compounds can be avoided which could cause negative effects during the activation of rhenium particles. Moreover, the rhenium (VII) sulfide particles obtained by the process of the invention have a defined particle size with a narrow particle size distribution.

To that extent, a further aspect of the invention relates to a composition comprising a plurality of particles comprising rhenium (VII) sulfide, wherein the particles comprising rhenium (VII) sulfide have a volume average particle size of 0.05 to 5 µm, a unimodal particle size distribution and a polydispersity Ð in the range of 1.0 to 2.5.

A further aspect of the invention concerns a composition as defined above, wherein the rhenium comprises the isotope rhenium-186 or the isotope rhenium-188. Such a composition is suitable for use as a medicament, in particular as a medicament for treating cancer, including, but not limited to skin cancer.

Thus, aspects of the present invention, including preferred embodiments thereof, are summarized in the following items.
1. A process for the preparation of particles comprising rhenium (VII) sulfide, which process comprises
   providing a reaction solution comprising perrhenic acid or a salt thereof, a Bronsted acid other than perrhenic acid and a sulfide source, and
   heating the reaction solution comprising perrhenic acid or a salt thereof, a Bronsted acid other than perrhenic acid, and a sulfide source by exposure to microwave radiation to convert the perrhenic acid or the salt thereof to particles comprising rhenium (VII) sulfide.
2. The process according to item 1, wherein the sulfide source is selected from a thioamide, Lawesson's reagent, a thiocarboxylate, phosphorous sulphide, (di)thiobisphthalimide, Belleau's reagent, Davy's reagent, Japanese reagent, hydrogen sulfide and a salt of hydrogen sulfide.
3. The process according to item 1, wherein the sulfide source is thioacetamide.
4. The process according to any of items 1 to 3, wherein the reaction solution comprising perrhenic acid or a salt thereof, a Bronsted acid other than perrhenic acid and a sulfide source further comprises a capping agent.
5. The process according to item 4, wherein the capping agent is selected from an organic polymer and a surfactant.
6. The process according to any of items 4 or 5, wherein the capping agent is polyvinylpyrrolidone.
7. The process according to any of items 1 to 6, wherein the reaction solution comprising perrhenic acid or a salt thereof, a Bronsted acid other than perrhenic acid, a sulfide source and optionally a capping agent, comprises water as a solvent.
8. The process according to any of items 1 to 7, wherein the reaction solution comprises an acid selected from nitric acid, sulfuric acid, chloric acid and perchloric acid as the Bronsted acid other than perrhenic acid.
9. The process according to item 8, wherein the reaction solution comprises nitric acid as the Bronsted acid other than perrhenic acid.
10. The process according to any of items 1 to 9, wherein providing the reaction solution comprising perrhenic acid or a salt thereof, a Bronsted acid other than perrhenic acid, a sulfide source and optionally a capping agent comprises the step of providing a solution comprising perrhenic acid or a salt thereof by dissolving and oxidizing metallic rhenium in a solution comprising an oxidizing agent.
11. The process according to item 10, wherein the oxidizing agent is a Bronsted acid selected from nitric acid, sulfuric acid, chloric acid and perchloric acid.
12. The process according to item 10, wherein the oxidizing agent is selected from a permanganate salt and hydroperoxide, and wherein providing the reaction solution comprising perrhenic acid or a salt thereof, a Bronsted acid other than perrhenic acid, a sulfide source and optionally a capping agent comprises the step of adding a Bronsted acid other than perrhenic acid to the solution comprising perrhenic acid or a salt thereof.
13. The process according to any of items 1 to 9, wherein the reaction solution comprises nitric acid as the Bronsted acid other than perrhenic acid, and wherein providing the reaction solution comprising perrhenic acid or a salt thereof, nitric acid, a sulfide source and optionally a capping agent comprises the steps of:
   (i) providing a solution comprising perrhenic acid and nitric acid by dissolving and oxidizing metallic rhenium in a solution comprising nitric acid, and
   (ii) combining the sulfide source and optionally the capping agent with the perrhenic acid and the nitric acid comprised in the solution provided in step (i) to provide a reaction solution comprising perrhenic acid, nitric acid, a sulfide source and optionally a capping agent.
14. The process according to item 13, wherein metallic rhenium is dissolved and oxidized in a solution comprising nitric acid while keeping the temperature of the solution at 95 °C or less, preferably 50 °C or less, more preferably 30 °C or less.
15. The process according to item 13 or 14, wherein the solution comprising nitric acid used to dissolve and oxidize the metallic rhenium in step (i) has a concentration of HNO₃ of 15 wt% or more, more preferably of 25 wt% to 45 wt%.
16. The process according to any of items 13 to 15, wherein the time between the quantitative dissolution of the metallic rhenium and the heating of the reaction solution is 30 minutes or less, more preferably 10 minutes or less.
17. The process according to any of items 13 to 16, which further comprises diluting the solution comprising the perrhenic acid and nitric acid obtained in step (i).
18. The process according to any of items 13 to 17, wherein the sulfide source and optionally the capping agent are combined with the perrhenic acid and the nitric acid comprised in the solution provided in step (i) without prior isolation of the perrhenic acid obtained by dissolving and oxidizing the metallic rhenium or of a salt thereof.
19. The process according to any of items 13 to 18, wherein the sulfide source and optionally the capping agent are combined with the perrhenic acid and the nitric acid comprised in the solution provided in step (i) without prior neutralization of the perrhenic acid obtained by dissolving and oxidizing the metallic rhenium.
20. The process according to any of items 1 to 9, wherein ammonium perrhenate is used as a salt of perrhenic acid to provide the reaction solution comprising perrhenic acid or a salt thereof, a Bronsted acid other than perrhenic acid, a sulfide source and optionally a capping agent.
21. The process according to any of items 1 to 20, wherein the reaction solution comprising perrhenic acid or a salt thereof, a Bronsted acid other than perrhenic acid, a sulfide source and optionally a capping agent, which is heated by exposure to microwave radiation, comprises the perrhenic acid or the salt thereof at a concentration of 0.1 mmol per litre to 40.0 mmol per litre, more preferably 1.0 mmol per litre to 10.0 mmol per litre, and still more preferably 1.0 mmol per litre to 6.0 mmol per litre, indicated as the molar amount of rhenium of the perrhenic acid or the salt thereof, per total volume of the reaction solution.
22. The process according to any of items 1 to 21, wherein the reaction solution comprising perrhenic acid or a salt thereof, a Bronsted acid other than perrhenic acid, a sulfide source and optionally a capping agent, which is heated by exposure to microwave radiation, comprises the Bronsted acid other than perrhenic acid at an equivalent concentration of 10 mmol per litre to 2 mol per litre, more preferably 40 to 1000 mmol per litre, still more preferably 40 to 200 mmol per litre, indicated as the molar amount of protons that can be provided by the Bronsted acid other than perrhenic acid, per total volume of the reaction solution.
23. The process according to any of items 1 to 22, wherein the reaction solution comprising perrhenic acid or a salt thereof, a Bronsted acid other than perrhenic acid, a sulfide source and optionally a capping agent, which is heated by exposure to microwave radiation, comprises a molar amount of Bronsted acid other than perrhenic acid which is at least three times, more preferably at least five times, and still more preferably at least ten times the molar amount of rhenium of the perrhenic acid or the salt thereof.
24. The process according to any of items 1 to 21, wherein the reaction solution comprises nitric acid as a Bronsted acid other than perrhenic acid, and wherein the reaction solution comprising perrhenic acid or a salt thereof, nitric acid, a sulfide source and optionally a capping agent, which is heated by exposure to microwave radiation, comprises the HNO₃ at a concentration of 10 mmol per litre to 2 mol per litre, more preferably 40 to 1000 mmol per litre, still more preferably 40 to 200 mmol per litre, indicated as the molar amount of HNO₃, per total volume of the reaction solution.
25. The process according to any of items 1 to 21 or 24, wherein the reaction solution comprises nitric acid as a Bronsted acid other than perrhenic acid, and wherein the reaction solution comprising perrhenic acid or a salt thereof, nitric acid, a sulfide source and optionally a capping agent, which is heated by exposure to microwave radiation, comprises a molar amount of HNO₃ which is at least three times, more preferably at least five times, and still more preferably at least ten times the molar amount of rhenium of the perrhenic acid or the salt thereof.
26. The process according to any of items 1 to 25, wherein the reaction solution comprising perrhenic acid or a salt thereof, a Bronsted acid other than perrhenic acid, a sulfide source and optionally a capping agent, which is heated by exposure to microwave radiation, comprises the sulfide source at a concentration of 10 mmol per litre to 2.1 mol per litre, more preferably 50 mmol per litre to 500 mmol per litre, and still more preferably 80 to 350 mmol per litre, indicated as the molar amount of sulfide that can be provided by the sulfide source, per total volume of the solution.
27. The process according to any of items 1 to 19, wherein the reaction solution comprising perrhenic acid or a salt thereof, a Bronsted acid other than perrhenic acid, a sulfide source and optionally a capping agent, which is heated by exposure to microwave radiation, comprises an equivalent molar amount of the sulfide source, indicated as the molar amount of sulfide that can be provided by the sulfide source, of at least 5 times, more preferably at least 10 times, more preferably at least 30 times, the molar amount of rhenium of the perrhenic acid or the salt thereof.
28. The process according to any of items 1 to 20, wherein the reaction solution comprising perrhenic acid or a salt thereof, a Bronsted acid other than perrhenic acid, a sulfide source and optionally a capping agent, which is heated by exposure to microwave radiation, comprises the capping agent at a concentration of 0 to 3.2 g per litre, more preferably 1.0 to 1.7 g per litre, indicated as the weight of the capping agent, per total volume of the solution.
29. The process according to any of items 1 to 28, wherein the reaction solution comprising perrhenic acid or a salt thereof, a Bronsted acid other than perrhenic acid, a sulfide source and optionally a capping agent is heated by the exposure to microwave radiation to a reaction temperature of 60°C to 150°C, more preferably 90 °C to 130 °C.
30. The process according to item 29, wherein the reaction solution is kept at the reaction temperature for a time of 1 minute to 5 hours, more preferably 10 to 30 minutes.
31. The process according to item 29 or 30, wherein the time span between the start of the exposure to microwave radiation and the time when the temperature of the reaction solution reaches the reaction temperature, is in the range of 0.1 to 30 minutes, more preferably in the range of 0.1 to 15 minutes.
32. The process according to any of items 1 to 31, wherein the heating rate of the reaction solution that is achieved by the exposure to microwave radiation is in the range of 1 °C/min to 100 °C/min, more preferably 4 °C/min to 30 °C/min.
33. The process according to any of items 1 to 32, wherein the reaction solution is stirred during the conversion of the perrhenic acid or the salt thereof to particles comprising rhenium (VII) sulfide.
34. The process according to any of items 1 to 33, wherein no alkali metal compounds are used for the preparation of the particles comprising rhenium (VII) sulfide.
35. The process according to any of items 1 to 34, wherein no halide compounds are used for the preparation of the particles comprising rhenium (VII) sulfide.
36. The process according to any of items 1 to 35, wherein the obtained particles comprising rhenium (VII) sulfide comprise the rhenium (VII) sulfide in an amount of 40 wt% or more, based on the total weight of the particles.
37. The process according to any of items 1 to 36, wherein the obtained particles comprising rhenium (VII) sulfide have a volume average particle size, which can be determined by dynamic light scattering, of 0.05 to 5 µm, more preferably 0.6 to 2.0 µm.
38. The process according to any of items 1 to 37, wherein the obtained particles comprising rhenium (VII) sulfide have a unimodal particle size distribution.
39. The process according to any of items 1 to 38, wherein the obtained rhenium (VII) sulfide particles have a particle size distribution with a polydispersity D in the range of 1.0 to 2.5, more preferably 1.0 to 2.0, and still more preferably 1.0 to 1.3, with Ð representing the ratio of the volume average particle size to the number average particle size as determined by dynamic light scattering.
40. The process according to any of items 1 to 39, wherein the process further comprises the irradiation of the obtained particles comprising rhenium (VII) sulfide with neutrons to convert rhenium-187 contained in the particles to rhenium-188 and/or to convert rhenium-185 contained in the particles to rhenium-186.
41. The process according to any of items 1 to 40, wherein the rhenium of the perrhenic acid or salt thereof comprised in the reaction solution comprising perrhenic acid or a salt thereof, nitric acid, a sulfide source and optionally a capping agent is enriched in the rhenium-187.
42. The process according to any of items 10 to 39, wherein the process further comprises, prior to step (i), the irradiation of the metallic rhenium with neutrons to convert rhenium-187 contained in the metallic rhenium to rhenium-188 and/or to convert rhenium-185 contained in the metallic rhenium to rhenium-186.
43. The process according to item 42, wherein the metallic rhenium, prior to its irradiation, with neutrons, is enriched in rhenium-187.
44. The process according to any of items 1 to 43, wherein at least 80 mol%, more preferably at least 90 mol%, still more preferably at least 95 mol%, and even more preferably at least 99 mol%, of all rhenium atoms in the particles comprising rhenium (VII) sulfide are rhenium-187 or rhenium-188 isotopes.
45. A process for the preparation of a composition comprising a plurality of particles comprising rhenium (VII) sulfide in a carrier material, said process comprising preparing particles comprising rhenium (VII) sulfide in accordance with the process of any of items 1 to 44, and incorporating the obtained rhenium (VII) sulfide particles into a carrier material.
46. The process according to item 45, wherein the carrier material is a fluid matrix.
47. The process according to item 45 or 46, wherein the carrier material is a polymer dispersion.
48. The process according to item 47, wherein the polymer dispersion is a dispersion of a (meth)acrylate polymer or a (meth)acrylate copolymer.
49. The process according to item 47 or 48, wherein the polymer dispersion is an aqueous dispersion.
50. The process according to any of items 45 to 49, wherein the carrier comprises TiO₂ particles as a filler.
51. The process according to any of items 45 to 50, wherein the process further comprises the irradiation of the particles comprising rhenium (VII) sulfide with neutrons after the incorporation into the carrier material to convert rhenium-187 contained in the particles to rhenium-188, and/or to convert rhenium-185 contained in the particles to rhenium-186.
52. A composition comprising a plurality of particles comprising rhenium (VII) sulfide, wherein the particles comprising rhenium (VII) sulfide have a volume average particle size, as determined by dynamic light scattering, of 0.05 to 5 µm, preferably 0.6 to 2 µm, and wherein the particles comprising rhenium (VII) sulfide have a unimodal particle size distribution and a polydispersity Ð in the range of 1.0 to 2.5, more preferably 1.0 to less than 2.0, and still more preferably 1.0 to 1.3, with Ð representing the ratio of the volume average particle size to the number average particle size as determined by dynamic light scattering.
53. The composition according to item 52, wherein the composition further comprises a carrier material into which the particles comprising rhenium (VII) sulfide are incorporated.
54. The composition according to item 53, wherein the carrier material is a fluid matrix.
55. The composition according to item 53 or 54, wherein the carrier material is a polymer dispersion.
56. The composition according to item 55, wherein the polymer dispersion is a dispersion of a (meth)acrylate polymer or a (meth)acrylate copolymer.
57. The composition according to item 55 or 56, wherein the polymer dispersion is an aqueous dispersion.
58. The composition according to any of items 53 to 57, wherein the carrier comprises TiO₂ particles as a filler.
59. The composition according to any of items 52 to 58, wherein the particles comprising rhenium (VII) sulfide are obtainable by the process according to any of items 1 to 44.
60. The composition according to any of items 52 to 59, which is obtainable by the process according to any of items 45 to 51.
61. The composition according to any one of items 52 to 60, wherein the particles comprising rhenium (VII) sulfide comprise rhenium-186 or rhenium-188, more preferably rhenium-188.
62. The composition according to item 61 for use in medicine.
63. The composition according to item 61 for use in the treatment of cancer.
64. The composition for use in accordance with item 63, wherein the cancer is skin cancer, preferably non-melanoma skin cancer, more preferably non-melanoma skin cancer selected from basal cell carcinoma and squamous cell carcinoma.
65. The composition for use in accordance with item 64, wherein the treatment comprises applying the composition to the affected skin area, preferably with a protective foil provided between the skin surface and the composition.
66. A method for the treatment of cancer, comprising applying a composition in accordance with item 61 to cancer tissue or administrating a composition in accordance with item 61 in the proximity of cancer tissue.
67. A method for the treatment of skin cancer, preferably non-melanoma skin cancer, more preferably non-melanoma skin cancer selected from basal cell carcinoma and squamous cell carcinoma, said method comprising applying the composition in accordance with item 61 to the affected skin area, preferably with a protective foil provided between the skin surface and the composition.
68. A method for the cosmetic treatment of skin, comprising applying a composition in accordance with item 61 to the skin, preferably with a protective foil provided between the skin surface and the composition.

As noted above, the process in accordance with the present invention comprises
providing a reaction solution comprising perrhenic acid or a salt thereof, a Bronsted acid other than perrhenic acid, a sulfide source, and optionally a capping agent, and
heating the reaction solution by exposure to microwave radiation to convert the perrhenic acid or the salt thereof to particles comprising rhenium (VII) sulfide (also referred to in the following as rhenium sulfide particles, or rhenium (VII) sulfide particles).

As will be understood by the skilled reader, reference is made herein to the solution comprising perrhenic acid or a salt thereof, a Bronsted acid other than perrhenic acid, a sulfide source and optionally a capping agent as a "reaction solution" since the perrhenic acid or the salt thereof reacts in the solution to form the rhenium (VII) sulfide. Heating of the reaction solution allows the reaction to proceed efficiently.

Rhenium (VII) sulfide contains the rhenium in its formal oxidation state +7. It can also be described by its chemical formula Re₂S₇.

Moreover, while the perrhenic acid or a salt thereof is contained in the reaction solution in dissolved form, it will be understood that it is not required for the solution that all of the components contained in the reaction solution are completely dissolved. Depending on the solubility and concentration of these components, they may e.g. be partly dispersed in the reaction solution.

As a rhenium source for the preparation of the rhenium (VII) sulfide particles, perrhenic acid (HReO₄) or a salt of perrhenic acid is contained in the reaction solution. As a salt of the perrhenic acid, a salt is preferably used which is free of alkali ions, such as ammonium perrhenate. However, it is preferred that the reaction solution contains perrhenic acid as a rhenium source.

Preferably, the reaction solution comprises an acid selected from nitric acid, sulfuric acid, chloric acid, perchloric acid and phosphoric acid as a Bronsted acid other than perrhenic acid. More preferably, it comprises an acid selected from nitric acid (HNO₃) or sulfuric acid (H₂SO₄). Still more preferably, the reaction solution comprises nitric acid as the Bronsted acid other than perrhenic acid, and most preferably the Bronsted acid other than perrhenic acid consists of nitric acid.

The provision of the reaction solution comprising perrhenic acid or a salt thereof, a Bronsted acid other than perrhenic acid, a sulfide source and optionally a capping agent may comprise the step of providing a solution comprising perrhenic acid or a salt thereof by dissolving and oxidizing metallic rhenium in a solution comprising an oxidizing agent.

Preferably, the oxidizing agent is a Bronsted acid selected from nitric acid, sulfuric acid, chloric acid and perchloric acid, more preferably a Bronsted acid selected from nitric acid and sulfuric acid, and most preferably nitric acid. As will be understood by the skilled reader, in cases where the oxidizing agent is a Bronsted acid, the Bronsted acid other than perrhenic acid contained in the reaction solution is preferably the same as the oxidizing agent.

Alternatively, the oxidizing agent can be selected from a permanganate salt and hydroperoxide. In this case, the oxidation is preferably carried out in an alkaline medium, and providing the reaction solution comprising perrhenic acid or a salt thereof, a Bronsted acid other than perrhenic acid, a sulfide source and optionally a capping agent preferably further comprises the step of adding a Bronsted acid other than perrhenic acid to the solution comprising perrhenic acid or a salt thereof.

As will be appreciated from the above, it is preferred that the reaction solution contains perrhenic acid as a rhenium source and is obtained by the dissolution and oxidation of metallic rhenium in a solution comprising nitric acid. In accordance with this preferred embodiment, the reaction solution is a reaction solution comprising perrhenic acid, nitric acid, a sulfide source and optionally a capping agent, and providing the reaction solution comprises the steps of:
(i) providing a solution comprising perrhenic acid and nitric acid by dissolving and oxidizing metallic rhenium in a solution comprising nitric acid, and
(ii) combining the sulfide source and optionally the capping agent with the perrhenic acid and the nitric acid comprised in the solution provided in step (i) to provide a reaction solution comprising perrhenic acid, nitric acid, a sulfide source and optionally a capping agent.

In accordance with this preferred embodiment, the nitric acid acts both as an oxidizing agent and as a Bronsted acid.

Metallic rhenium can be advantageously used for dissolution, such as the dissolution in step (i) of the above preferred embodiment, e.g. in the form of a metal powder, or in the form of metal chips.

The solvent for the solution wherein the metallic rhenium is dissolved, e.g. the solution comprising nitric acid of step (i) above, is generally selected from an organic protic solvent, such as an alcohol, and water. It is preferred for economic and environmental reasons that the solution comprises water as the solvent, and it is further preferred that water is a main solvent with an amount of 50 vol% or more, based on the total volume of solvent as 100 vol%. It is most preferred that water is the only solvent used in the solution. Unless specified otherwise, volumes and volume percentages referred to herein are indicated for a reference temperature of 25°C and a reference pressure of 100 kPa.

The concentration of the nitric acid in the solution comprising nitric acid used in step (i) to dissolve and oxidize the metallic rhenium should be sufficiently high that the dissolution and the oxidation of the metallic rhenium proceeds at a high rate. Preferably, the concentration of the nitric acid is 15 wt% or more, more preferably 25 wt% or more, based on the total weight of the solution containing nitric acid, prior to the addition of the metallic rhenium, as 100 wt%. Since the reaction of the metallic rhenium with the nitric acid is exothermic, the control of the reaction can be facilitated if the concentration of the nitric acid is kept at 65 wt% or less, more preferably at 45 wt% or less, based on the total weight of the solution containing nitric acid, prior to the addition of the metallic rhenium, as 100 wt%. Thus, a particularly preferred solution comprising nitric acid to be used in step (i) above is a solution comprising 25 to 45 wt% of nitric acid in water.

In order to provide the solution comprising perrhenic acid and nitric acid in step (i), the HNO₃ is typically used in a significant excess in terms of its molar amount compared to the molar amount of rhenium to be dissolved in the solution.

The dissolution and oxidation of the metallic rhenium in a solution comprising an oxidizing agent such as nitric acid can be conveniently accomplished by combining the metallic rhenium with a solution comprising the oxidizing agent, e.g. by adding the metallic rhenium to a solution comprising nitric acid or by adding the solution comprising nitric acid to the metallic rhenium. Typically, the solution is agitated, e.g. stirred, while the metallic rhenium is dissolved. Preferably, a suspension of rhenium metal in water is stirred before addition of nitric acid of higher concentration. This addition can be conducted in one portion or, more preferably in multiple dosing steps to ensure a safe process.

It is preferred that the metallic rhenium is dissolved and oxidized in a solution comprising an oxidizing agent such as nitric acid while keeping the temperature of the solution at 95 °C or less, preferably 40 °C or less and most preferably 30 °C or less. Thus, a loss of volatile perrhenic acid can be minimized, and the yield of perrhenic acid in the reaction solution can be optimized.

It is preferred that the time between the quantitative dissolution of the metallic rhenium and the heating of the reaction solution comprising perrhenic acid, the Bronsted acid such as nitric acid, a sulfide source and optionally a capping agent by the exposure to microwave radiation is 30 minutes or less, more preferably 10 minutes or less. On this preferred timescale, a loss of volatile perrhenic acid can be minimized while the dissolution and oxidation can proceed in a satisfactory manner.

If the reaction solution is a reaction solution comprising perrhenic acid, and providing the reaction solution comprises steps (i) and (ii) discussed above, the process in accordance with the invention may further comprise diluting the solution comprising the perrhenic acid and nitric acid obtained in step (i), e.g. prior to combining the sulfide source and optionally the capping agent with the perrhenic acid and the nitric acid comprised in the solution provided in step (i). As will be understood by the skilled reader, such a dilution is part of the claimed process, it is generally accomplished by adding a solvent to the solution obtained in step (i), and the solvent used for the dilution can be, e.g., a solvent as it is present in the solution containing the nitric acid used in step (i). Thus, the solvent used or the dilution is generally selected from an organic protic solvent, such as an alcohol, and water, and it is preferred for economic and environmental reasons that the solvent used for the dilution comprises water. It is further preferred that water is a main solvent with an amount of 50 vol% or more, based on the total volume of solvent as 100 vol%. It is most preferred that water is the only solvent used for diluting the solution obtained in step (i).

In step (ii) as discussed above, the sulfide source and optionally the capping agent are combined with the perrhenic acid and the nitric acid comprised in the solution provided in step (i). It is preferred that the sulfide source and optionally the capping agent are combined with the perrhenic acid and the nitric acid comprised in the solution provided in step (i) without prior neutralization of the perrhenic acid obtained by dissolving and oxidizing the metallic rhenium. It is also preferred that these components are combined without prior isolation of the perrhenic acid obtained by dissolving and oxidizing the metallic rhenium from the solution obtained in step (i), and also without the isolation of a salt of the perrhenic acid as it could result from a neutralization step. In other words, it is preferred that the perrhenic acid provided in step (i) is combined in step (ii) with the sulfide source and optionally the capping agent without previous neutralization and/or isolation thereof from the solution provided in step (i).

However, if the preferred variant comprising steps (i) and (ii) is not used, it is also possible to combine the perrhenic acid or a salt thereof, the Bronsted acid such as nitric acid, the sulfide source and optionally the capping agent in a solvent in any other order in order to provide the reaction solution comprising perrhenic acid or a salt thereof, Bronsted acid such as nitric acid, a sulfide source, and optionally a capping agent. The following explanations regarding the reaction solution and its components thus apply for reaction solutions provided using the preferred variant comprising steps (i) and (ii) discussed above, as well as for reaction solution prepared in another way, e.g. using ammonium perrhenate as a salt of perrhenic acid to provide the reaction solution. Unless indicated otherwise, concentrations or molar ratios of component contained in the reaction solution are generally indicated for the reaction solution at the onset of the heating by exposure to microwave radiation. As will be appreciated by the skilled person, while the reaction proceeds in the heated reaction solution, the concentrations and/or ratios may be subject to changes. Moreover, as noted above, volumes and volume percentages referred to herein are generally indicated for a reference temperature of 25°C and a reference pressure of 100 kPa.

The reaction solution comprising perrhenic acid or a salt thereof, a Bronsted acid such as nitric acid, a sulfide source and optionally a capping agent, which is heated by exposure to microwave radiation, preferably comprises the perrhenic acid or the salt thereof at a concentration of 0.1 mmol per litre to 40.0 mmol per litre, more preferably 1.0 mmol per litre to 10.0 mmol per litre, and still more preferably 1.0 mmol per litre to 6.0 mmol per litre, indicated as the molar amount of rhenium of the perrhenic acid or the salt thereof, per total volume of the reaction solution.

The reaction solution comprising perrhenic acid or a salt thereof, a Bronsted acid other than perrhenic acid, a sulfide source and optionally a capping agent, which is heated by exposure to microwave radiation, preferably comprises the Bronsted acid at an equivalent concentration of 10 mmol per litre to 2 mol per litre, more preferably 40 to 1000 mmol per litre, and still more preferably 40 to 200 mmol per litre. As will be understood by the skilled reader, the equivalent concentration represents the molar concentration of the Bronsted acid divided by an equivalence factor f_{eq} which reflects the number of protons that can be provided per one molecule of the Bronsted acid. For a monoprotic acid, such as nitric acid, f_{eq} is 1 or 1/ f_{eq} is 1, for a diprotic acid, such as H₂SO₄, 1/ f_{eq} is 2, and for a triprotic acid 1/ f_{eq} is 3. Thus, the equivalent concentration is indicated as the molar amount that can be provided by the Bronsted acid, per total volume of the reaction solution.

Thus, in accordance with the preferred embodiment where the reaction solution comprises nitric acid as the Bronsted acid other than perrhenic acid, the reaction solution comprising perrhenic acid or a salt thereof, nitric acid, a sulfide source and optionally a capping agent, which is heated by exposure to microwave radiation, preferably comprises HNO₃ at a concentration of 10 mmol per litre to 2 mol per litre, more preferably 40 to 1000 mmol per litre, and still more preferably 40 to 200 mmol per litre, indicated as the molar amount of HNO₃, per total volume of the reaction solution.

The molar amount of the Bronsted acid other than perrhenic acid in the reaction solution comprising perrhenic acid or a salt thereof, the Bronsted acid, a sulfide source and optionally a capping agent, which is heated by exposure to microwave radiation, is preferably at least three times, more preferably at least five times, and still more preferably at least ten times the molar amount of rhenium of the perrhenic acid or the salt thereof.

Thus, in accordance with the preferred embodiment where the reaction solution comprises nitric acid as the Bronsted acid other than perrhenic acid, the molar amount of HNO₃ in the reaction solution comprising perrhenic acid or a salt thereof, nitric acid, a sulfide source and optionally a capping agent, which is heated by exposure to microwave radiation, is preferably at least three times, more preferably at least five times, and still more preferably at least ten times the molar amount of rhenium of the perrhenic acid or the salt thereof.

The sulfide source contained in the reaction solution is preferably selected from a thioamide, Lawesson's reagent (2,4-Bis(4-methoxyphenyl)-1,3,2,4-dithiadiphosphetane-2,4-dithione), a thiocarboxylate, phosphorous sulphide, (di)thiobisphthalimide, Belleau's reagent (2,4-Bis(4-phenoxyphenyl)-1,3-dithia-2,4-diphosphetane-2,4-disulfide), Davy's reagent (e.g. 2,4-Bis(p-tolylhio)-1,3,2,4-dithiadiphosphetane-2,4-disulfide), Japanese reagent (2,4-Bis(phenylthio)-2,4-dithioxo-1,3,2,4-dithiadiphosphetane), hydrogen sulfide, and a salt of hydrogen sulfide. Among these, a thioamide is preferred, and most preferably, the sulfide source is thioacetamide. If hydrogen sulfide is used as a sulfide source in the reaction solution, it is expedient to provide the reaction solution and to heat the reaction solution in an atmosphere comprising H₂S in a gas tight reactor, preferably a gas tight reactor wherein pressure can be applied to the reaction solution.

The reaction solution which is heated by exposure to microwave radiation preferably comprises the sulfide source at a concentration of 10 mmol per litre to 2.1 mol per litre, more preferably 50 mmol per litre to 500 mmol per litre, and still more preferably 80 to 350 mmol per litre, indicated as the molar amount of sulfide ions that can be provided by the sulfide source, per total volume of the solution. In consideration of the structure of the compound used as a sulfide source, the skilled reader will be able to determine the amount of sulfide ions that that can be provided by the sulfide source without problems. For example, a thioamide, such as thioacetamide, can provide one mol of sulfide ions per mol of the compound acting as the sulfide source. Thus, if a thioamide is used as the sulfur source, the reaction solution preferably comprises the thioamide at a concentration of 10 mmol per litre to 2.1 mol per litre, more preferably 50 mmol per litre to 500 mmol per litre, and still more preferably 80 to 350 mmol per litre, indicated as the molar amount of sulfide ions that can be provided by the sulfide source, per total volume of the solution.

The equivalent molar amount of the sulfide source comprised by the reaction solution which is heated by exposure to microwave radiation, indicated as the molar amount of sulfide that can be provided by the sulfide source, is preferably at least 5 times, more preferably at least 10 times, more preferably at least 30 times, the molar amount of rhenium of the perrhenic acid or the salt thereof.

The capping agent, i.e. a substance which is able to adhere to the surface of the rhenium (VII) sulfide particles formed by the process in accordance with the present invention and helps to prevent the agglomeration of these particles, is preferably contained in the reaction solution together with the perrhenic acid or a salt thereof, the Bronsted acid other than perrhenic acid, such as nitric acid, and the sulfide source.

Typically, an organic polymer or a surfactant is used as a capping agent.

Suitable organic polymers preferably have flexible polymer chains wherein either sterically demanding side chains (such as long alkyl groups) or polar functional groups are provided either as constituents of the polymer backbone (i.e. as constituents of the main chain) or as constituents of possible side chains. Suitable polar functional groups can be selected e.g. from an alcohol, ether, ester, carbonyl, amide, carboxylate or carboxylic acid, imide, lactone, lactame, carbonate, urethane, urea, amine, ammonium group, phosphor containing group, sulfide, sulfone, sulphite and sulfonate. Examples of organic polymers suitable as a capping agent are poly (vinyl pyrrolidone), poly (acrylates), poly (ethylene glycol), poly (vinyl alcohol) or poly (vinyl pyrrolidine).

According to aforementioned definition, organic polymers can also be bio-based (e.g. BSA, polyphenols, enzymes, citric acid, vitamins).

Surfactants which can be used as capping agents include ionic and non-ionic surfactants, singly or in combination. Ionic and non-ionic surfactants may comprise O-, P-, N- or S-containing functional groups, e.g. the exemplary polar functional groups listed for the organic polymers above. Examples of ionic or non-ionic surfactants suitable as a capping agent are sodium dodecyl sulphate, cetyltrimethyl ammonium bromide, oleic acid or comparable salts of salt-forming functional groups listed for the organic polymers above.

Preferably, the capping agent is polyvinylpyrrolidone (PVP), e.g. PVP with a weight average molecular weight in the range of 1,000 to 500,000 g/mol, more preferably PVP with a weight average molecular weight in the range of 40,000 to 60,000 g/mol. The weight average molecular weight can be determined, e.g., via size-exclusion-chromatography, such as gel permeation chromatography (GPC).

The reaction solution which is heated by exposure to microwave radiation, preferably comprises the capping agent, e.g. the PVP, and particularly preferably the PVP with a weight average molecular weight in the range of 40,000 to 60,000 g/mol, at a concentration of 0 to 3.2 g per litre, more preferably 1.0 to 1.7 g per litre, indicated as the weight of the capping agent per total volume of the solution.

The solvent of the reaction solution which is heated by exposure to microwave radiation is generally a solvent selected from an organic protic solvent, such as an alcohol, and water. It is preferred for economic and environmental reasons that the solution comprises water as the solvent, and it is further preferred that water is a main solvent with an amount of 50 vol% or more, based on the total volume of solvent as 100 vol%. It is most preferred that water is the only solvent used in the solution.

If desired, further components may be incorporated into the reaction solution. However, it is generally not necessary that such further components be used in the reaction solution, in particular the reaction solution at the onset of heating, may essentially consist of, or may consist of the solvent, perrhenic acid or a salt thereof, a Bronsted acid other than perrhenic acid, such as nitric acid, a sulfide source and optionally a capping agent. In this context, the term "essentially consist of" means that other components than the ones listed account for a maximum of 5 wt%, preferably a maximum of 2 wt%, of the reaction solution, based on the total weight of the reaction solution as 100 wt%.

It is one advantage of the process in accordance with the invention that neither compounds containing alkali metals, such as sodium hydroxide for neutralization, nor compounds containing halides, such as HCI for acidification, are required in order to provide the rhenium (VII) sulfide particles. Both alkali metals and halides can be disadvantageous as impurities if the rhenium (VII) sulfide particles are activated via irradiation with neutrons, since they tend to form radioactive isotopes with problematic half-lifes and/or undesirable forms of radiation. Thus, it is preferred that no alkali metal compounds are used for the preparation of the rhenium (VII) sulfide particles. Likewise, it is preferred that no halide compounds are used for the preparation of the rhenium (VII) sulfide particles.

In line with the above, it will be understood that a preferred variant of the present invention relates to a process for the preparation of particles comprising rhenium (VII) sulfide, which process comprises
providing a reaction solution comprising or consisting of water, perrhenic acid or a salt thereof, nitric acid, thioacetamide, and polyvinylpyrrolidone, and
heating the reaction solution comprising or consisting of perrhenic acid or a salt thereof, nitric acid, and a sulfide source by exposure to microwave radiation to convert the perrhenic acid or the salt thereof to particles comprising rhenium (VII) sulfide. As noted above, the thioacetamide is suitable as a sulfide source, while the PVP is suitable as a capping agent.

Moreover, a still more preferred variant of the present invention relates to a process for the preparation of particles comprising rhenium (VII) sulfide, which process comprises
(i) providing a solution comprising or consisting of water, perrhenic acid and nitric acid by dissolving and oxidizing metallic rhenium in a solution comprising or consisting of water and nitric acid, and
(ii) combining the thioacetamide and the polyvinylpyrrolidone with the perrhenic acid and the nitric acid comprised in the solution provided in step (i) to provide a reaction solution comprising or consisting of water, perrhenic acid, nitric acid, thioacetamide and polyvinylpyrrolidone; and
heating the reaction solution comprising or consisting of water, perrhenic acid, nitric acid, thioacetamide and polyvinylpyrrolidone by exposure to microwave radiation to convert the perrhenic acid to particles comprising rhenium (VII) sulfide.

In the process in accordance with the invention, the reaction solution is heated by exposure to microwave radiation. It has been found that the input of energy via microwaves allows the solution to be heated quickly, resulting in a favourably narrow particle size distribution of the obtained rhenium (VII) sulfide particles. Preferably, the reaction solution comprising perrhenic acid or a salt thereof, the Bronsted acid other than perrhenic acid such as nitric acid, a sulfide source and optionally a capping agent is heated by the exposure to microwave radiation to a reaction temperature of 60°C to 150°C, more preferably 90 °C to 130 °C.

Typically, the reaction solution is kept at the reaction temperature where the perrhenic acid or the salt thereof is converted to rhenium (VII) sulfide particles, preferably at a reaction temperature within the preferred temperature range indicated above and more preferably at a reaction temperature within the more preferred temperature range indicated above, for a time of 1 minute to 5 hours, more preferably 10 to 30 minutes.

The time for heating the mixture to the reaction temperature (i.e. the time span between the start of the exposure to microwave radiation and the time when the temperature of the reaction solution reaches the reaction temperature, also referred to as "ramp time"), is preferably in the range of 0.1 to 30 minutes, more preferably in the range of 0.1 to 15 minutes.

High heating rates may be beneficial in order to optimize the particle size distribution of the obtained rhenium (VII) sulfide particles, so that typically a maximum heating rate is used. Preferably, the heating rate is of the reaction solution that is achieved by the exposure to microwave radiation is in the range of 1 °C/min to 100 °C/min, more preferably 4 °C/min to 30 °C/min.

While the reaction solution is kept at the reaction temperature, the perrhenic acid or the salt thereof is converted to rhenium (VII) sulfide particles, and the rhenium (VII) sulfide particles typically precipitate from the solution.

Preferably, the reaction solution is agitated, e.g. stirred, during the conversion of the perrhenic acid or the salt thereof to rhenium (VII) sulfide particles.

In order to expose the reaction solution to microwave radiation, a conventional microwave reactor can be used. To efficiently heat the reaction solution, the wavelength of the microwaves to which the reaction solution is exposed is typically in the range of 1 mm to 30 cm. As will be understood by the skilled person, the wavelength is such that the microwaves are absorbed by components, in particular by the solvent, of the reaction solution. The power output of the microwave reactor is not particularly limited. As will be understood by the skilled person, the power output is preferably sufficient to allow the preferred ramp times indicated above to be achieved. For example, microwave reactors with a power output in the range of 100 W to 2000 W can be used.

The volume of the reaction solution which is exposed to the microwave radiation is typically adapted to the microwave reactor which is used. If larger volumes of a reaction solution are to be heated, it may be advantageous to separate larger amounts of reaction solution into multiple reaction compartments or reaction vessels in a microwave reactor.

After the reaction solution has been kept at the reaction temperature for a desired period of time, and the conversion of the perrhenic acid or the salt thereof to rhenium (VII) sulfide particles has been allowed to proceed, the resulting solution comprising the rhenium (VII) sulfide particles is preferably cooled. The rhenium (VII) sulfide particles can be isolated from the solution using known means, such as decantation, filtration or centrifugation. The isolated particles can be washed, e.g. with water, and/or dried.

The particles comprising rhenium (VII) sulfide obtained by the process in accordance with the invention typically comprise rhenium (VII) sulfide in an amount of more than 30 wt%, preferably more than 50 wt%, and more preferably more than 60 wt%, based on the total weight of the particles. As an additional component, the rhenium (VII) sulfide particles may comprise e.g. a capping agent as defined above, such as PVP.

Preferably, the obtained rhenium (VII) sulfide particles have a volume average particle size of 0.05 to 5 µm, more preferably 0.6 to 2.0 µm.

The particle size distribution of the rhenium (VII) sulfide particles is preferably a unimodal particle size distribution.

As regards the breadth of the particle size distribution, it is preferred that the obtained rhenium (VII) sulfide particles have a particle size distribution with a polydispersity Ð in the range of 1.0 to 2.5, more preferably 1.0 to 2.0, and still more preferably 1.0 to 1.3, with Ð representing the ratio of the volume average particle size to the number average particle size.

In this regard, it has been found that heating the reaction solution by exposure to microwave radiation allows shorter reaction times and steeper temperature ramps to be used, and a narrower particle size distribution to be achieved.

For example, using steeper temperature ramps has been found to be efficient to achieve a narrower particle size distribution. Furthermore, optimization of the particle size and particle size distribution is possible by adjusting the concentration of the perrhenic acid, of the Bronsted acid other than perrhenic acid such as nitric acid, and/or of the optional capping agent in the reaction solution, especially within the preferred concentration ranges indicated above.

The volume average particle size, the number average particle size and the shape of the particle size distribution can be determined via dynamic light scattering (DLS), using a dispersion of the particles in water with a temperature of 25°C as a sample, e.g. with a scattering angle of 173° and a laser wavelength of 633 nm.

Thus, as will be apparent from the above, it is particularly preferred that the rhenium (VII) sulfide particles provided by the process in accordance with the invention have a volume average particle size of 0.05 to 5 µm, preferably 0.6 to 2 µm, and that they have a unimodal particle size distribution and a polydispersity D in the range of 1.0 to 2.5, more preferably 1.0 to 2.0, and still more preferably 1.0 to 1.3, with D representing the ratio of the volume average particle size to the number average particle size.

It is known that large particles containing radioisotopes may create local hot spots in a composition which is applied in epidermal radioisotope therapy, giving rise to burns on the skin of a patient. On the other hand, a large fraction of small particles may increase the loss of the rhenium compound during the production and the isolation of the particles, and during their further formulation. Thus, a defined particle size and a narrow particle size distribution as they can be obtained in the context of the present invention are desirable.

In order to facilitate the handling or the application of the rhenium (VII) sulfide particles, e.g. in radioisotope therapy, the particles are preferably provided as a composition comprising a plurality of the particles comprising rhenium (VII) sulfide in a carrier material. Thus, the present invention further encompasses a process for the preparation of a composition comprising a plurality of particles comprising rhenium (VII) sulfide in a carrier material, said process comprising preparing particles comprising rhenium (VII) sulfide in accordance with the process described above, and incorporating the obtained particles comprising rhenium (VII) sulfide into a carrier material. Typically, the particles comprising rhenium (VII) sulfide are dispersed in the carrier material.

The carrier material is preferably a fluid matrix. Moreover, it is preferred that the carrier material is a polymer dispersion, i.e. a dispersion wherein a polymer material is dispersed in a liquid phase. Preferred as the polymer of the polymer dispersion is a (meth)acrylate polymer or a (meth)acrylate copolymer. As will be understood by the skilled person, the reference to "(meth)acrylate" (co)polymer encompasses (co)polymers formed from methacrylate monomers and/or acrylate monomers. The polymer dispersion is preferably an aqueous dispersion, i.e. a dispersion using water in the liquid phase. The liquid phase of the polymer dispersion typically comprises water as a main solvent with an amount of 50 vol% or more, based on the total volume of the liquid phase as 100 vol%. It is most preferred that water is the only solvent used in the liquid phase. Unless specified otherwise, volumes and volume percentages referred to herein are indicated for a reference temperature of 25°C and a reference pressure of 100 kPa. The carrier material may comprise a filler, e.g. TiO₂ particles, in addition to the rhenium (VII) sulfide particles.

For the composition comprising a plurality of the particles comprising rhenium (VII) sulfide in a carrier material in accordance with the invention, it is preferred that the particles further comprise the capping agent as discussed above. The capping agent may improve the dispersibility of the particles in the carrier material.

Thus, particles comprising rhenium (VII) sulfide and a capping agent obtained from the preferred process of preparation using a capping agent, such as PVP, may show increased dispersibility in a carrier material, preferably in a matrix provided by a carrier material comprising a (meth)acrylate polymer or a (meth)acrylate copolymer as organic polymer and titanium dioxide as preferred filler component preferentially with a negative surface charge.

The process for the preparation of the composition comprising the plurality of rhenium (VII) sulfide particles in a carrier material preferably comprises
preparing rhenium (VII) sulfide particles in accordance with the process described above, and
incorporating the obtained rhenium (VII) sulfide particles into a carrier material by providing a suspension of the rhenium (VII) sulfide particles in a solvent, and mixing the suspension with the carrier material, optionally followed by a removal of the solvent used to provide the suspension of the rhenium (VII) sulfide particles. As a solvent to suspend the rhenium (VII) sulfide particles, e.g. ethanol can be used.

In order to be used as an active material in radioisotope therapy, the rhenium (VII) sulfide particles prepared in accordance with the process of the invention or the composition prepared in accordance with the process of the invention may contain a radioisotope of rhenium. As such a radioisotope, the radioisotope rhenium-186 and the radioisotope rhenium-188 are particularly suitable for radioisotope therapy in terms of their emitted radiation and in terms of their half-life. Rhenium-188 is preferred as a radioisotope for radioisotope therapy, so that the rhenium (VII) sulfide particles and the composition prepared in accordance with the processes of the invention preferably contain rhenium-188.

The radioisotope rhenium-186 may be obtained via irradiation of the isotope rhenium-185 in the form of a metal or in the form of a compound thereof (e.g. rhenium (VII) sulfide) with neutrons. Likewise, the radioisotope rhenium-188 may be obtained via irradiation of the isotope rhenium-187 in the form of a metal or in the form of a compound thereof (e.g. rhenium (VII) sulfide) with neutrons.

To that extent, the processes of the present invention for the preparation of rhenium sulfide particles and for the preparation of a composition comprising these particles may comprise a step of irradiating rhenium in the form of metallic rhenium, or rhenium contained in a rhenium compound such as the perrhenic acid or the salt thereof, or the rhenium (VII) sulfide of the rhenium (VII) sulfide particles, with neutrons, to convert rhenium-185 contained therein to rhenium-186 and/or to convert rhenium-187 contained therein to rhenium-188. Since rhenium-188 is preferred as a radioisotope in the rhenium (VII) sulfide particles, the metallic rhenium or the rhenium compound which is irradiated with neutrons is preferably enriched in rhenium-187, i.e. its content of rhenium-187 is higher than 63 mol%. Preferably, the content of rhenium-187 in the metallic rhenium or the rhenium compound enriched with rhenium-187 is at least 80 mol%, more preferably at least 90 mol%, still more preferably at least 95 mol%, and even more preferably at least 99 mol%, based on the total amount of rhenium atoms.

Thus, it is equally preferred that at least 80 mol%, more preferably at least 90 mol%, still more preferably at least 95 mol%, and even more preferably at least 99 mol%, of all rhenium atoms in the rhenium (VII) sulfide particles are rhenium-187 or rhenium-188 isotopes.

Preferably, the processes of the present invention comprise one of the following procedures to provide rhenium (VII) sulfide particles comprising a radioisotope.
a) A step of irradiating the rhenium (VII) sulfide particles, preferably rhenium (VII) sulfide particles enriched with rhenium-187, which are obtained from the conversion of the perrhenic acid or the salt thereof to rhenium (VII) sulfide particles in the process in accordance with the present invention, with neutrons, to obtain rhenium (VII) sulfide particles comprising a rhenium radioisotope, preferably rhenium-188.
   The irradiation of rhenium (VII) sulfide particles is preferably carried out using isolated rhenium (VII) sulfide particles, i.e. if a composition comprising a plurality of rhenium (VII) sulfide particles in a carrier material is prepared by the process in accordance with the invention, the irradiation is typically carried out prior to the incorporation of the rhenium (VII) sulfide particles into the carrier material.
b) A step of irradiating metallic rhenium, preferably metallic rhenium enriched with rhenium-187, with neutrons to obtain metallic rhenium comprising a rhenium radioisotope, preferably rhenium-188, and providing the solution comprising perrhenic acid and nitric acid by dissolving and oxidizing the irradiated metallic rhenium in a solution comprising nitric acid in line with the preferred variant of the process for the preparation of rhenium (VII) sulfide particles discussed above.

As noted above, the invention encompasses, as a further aspect, a composition comprising a plurality of rhenium (VII) sulfide particles, wherein the rhenium (VII) sulfide particles have a volume average particle size, as determined by dynamic light scattering, of 0.05 to 5 µm, preferably 0.6 to 2 µm, and wherein the rhenium (VII) sulfide particles have a unimodal particle size distribution and a polydispersity D in the range of 1.0 to 2.5, more preferably 1.0 to less than 2.0, and still more preferably 1.0 to 1.3, with Ð representing the ratio of the volume average particle size to the number average particle size as determined by dynamic light scattering.

As indicated above, the measurements using dynamic light scattering (DLS) are typically carried out on a dispersion of the particles in water with a temperature of 25°C as a sample, e.g. with a scattering angle of 173° and a laser wavelength of 633 nm.

As will be understood by the skilled reader, such a composition can be conveniently obtained by the process for the preparation of rhenium (VII) sulfide particles in accordance with the present invention. Thus, unless indicated otherwise, the information provided with regard to the rhenium (VII) sulfide particles resulting from the process of the present invention equally applies for the rhenium (VII) sulfide particles of the above composition.

Moreover, the composition in accordance with the invention may comprise the rhenium (VII) sulfide particles in a carrier material, as it is discussed above with regard to the process variant of the present invention related to the process for the preparation of a composition comprising a plurality of rhenium (VII) sulfide particles in a carrier material. The information provided with regard to the carrier material in the context of this process likewise applies for the carrier material which may be present in the composition in accordance with the invention. If the composition comprises such a carrier material, the rhenium (VII) sulfide particles are typically dispersed therein. It will be understood that the composition in accordance with the invention comprising the rhenium (VII) sulfide particles as defined above in a carrier material, can be obtained by the process variant of the present invention related to the process for the preparation of a composition comprising a plurality of rhenium (VII) sulfide particles in a carrier material.

As explained above, the carrier material is preferably a fluid matrix. Moreover, it is preferred that the carrier material is a polymer dispersion, i.e. a dispersion wherein a polymer material is dispersed in a liquid phase. Preferred as the polymer of the polymer dispersion is a (meth)acrylate polymer or a (meth)acrylate copolymer. As will be understood by the skilled person, the reference to "(meth)acrylate" (co)polymer encompasses (co)polymers formed from methacrylate monomers and/or acrylate monomers. The polymer dispersion is preferably an aqueous dispersion, i.e. a dispersion using water in the liquid phase. The liquid phase of the polymer dispersion typically comprises water is a main solvent with an amount of 50 vol% or more, based on the total volume of the liquid phase as 100 vol%. It is most preferred that water is the only solvent used in the liquid phase. Unless specified otherwise, volumes and volume percentages referred to herein are indicated for a reference temperature of 25°C and a reference pressure of 100 kPa. The carrier material may comprise a filler, e.g. TiO₂ particles, in addition to the rhenium (VII) sulfide particles.

Moreover, as noted above, the rhenium (VII) sulfide particles may contain a radioisotope, in particular rhenium-186 and/or rhenium-188. As pointed out in the discussion of the process in accordance with the invention, it is preferred that at least 80 mol%, more preferably at least 90 mol%, still more preferably at least 95 mol%, and even more preferably at least 99 mol%, of all rhenium atoms in the rhenium (VII) sulfide particles are rhenium-187 isotopes or rhenium-188 isotopes. The rhenium-187 isotopes can be converted to rhenium-188 isotopes via irradiation with neutrons.

A composition in accordance with the invention wherein the rhenium (VII) sulfide particles comprise a radioisotope of rhenium, in particular rhenium-186 and/or rhenium-188, and more preferably rhenium-188, is further provided for use in medicine or for use in the treatment of the human or animal body by therapy.

The composition comprising a radioisotope of rhenium is preferably provided for use in the treatment of cancer. For this purpose, it can be applied to cancer tissue or administrated in the proximity of cancer tissue. As will be understood by the skilled reader, "in the proximity" refers in this context to a distance which allows the radioactive rays (beta rays) emitted by the radioisotopes of the rhenium to reach the cancer tissue.

A type of cancer which is preferably treated using the compositions in accordance with the invention is skin cancer, more preferably non-melanoma skin cancer, and still more preferably non-melanoma skin cancer selected from basal cell carcinoma and squamous cell carcinoma.

The treatment of skin cancer preferably comprises applying the composition to the affected skin area. It has been found advantageous to use a protective foil for the application which is provided between the skin surface and the composition. As will be understood by the skilled person, such a protective foil may be used to prevent a direct contact of the composition and its constituents with the skin, but it allows the radioactive rays (beta rays) of emitted by the radioisotopes of the rhenium to reach the cancer tissue.

In line with the above, the composition in accordance with the invention wherein the rhenium (VII) sulfide particles comprise a radioisotope of rhenium, in particular rhenium-186 and/or rhenium-188, and more preferably rhenium-188, can be used in a method for the treatment of cancer, said method comprising applying the composition to cancer tissue or administrating the composition in the proximity of cancer tissue.

Likewise, the composition can be used in a method for the treatment of skin cancer, preferably non-melanoma skin cancer, more preferably non-melanoma skin cancer selected from basal cell carcinoma and squamous cell carcinoma, said method comprising applying the composition to the affected skin area, preferably with a protective foil provided between the skin surface and the composition.

Moreover, a composition in accordance with the invention wherein the rhenium (VII) sulfide particles comprise a radioisotope of rhenium, in particular rhenium-186 and/or rhenium-188, and more preferably rhenium-188, can be used in a method for the cosmetic (i.e. non-therapeutic) treatment of the skin, said method comprising applying the composition to the skin, preferably with a protective foil provided between the skin surface and the composition.

The following examples serve to illustrate the invention.

### Examples

For the preparation of rhenium sulfide particles, large-scale microwave synthesis (A) and small-scale microwave synthesis (B) is illustrated in the following. As a comparative example, rhenium sulfide particles are produced using conventional heating (C) in accordance with conventionally heated syntheses.

Perrhenic acid or a salt thereof was provided as ammonium perrhenate or via dissolution of metallic rhenium in nitric acid and use of the resulting mixture in the microwave (MW) assisted conversion towards rhenium sulfide particles.

First, general experimental details are described in the following section "General Information" which are valid for all preparation methods, followed by specific details of the methods (A), (B) and (C).

### 1. General Information

### Tools, Reactors and Glassware

Solutions were dosed with Eppendorf pipettes research plus (10-100 µL; 2-20µL) and reference (100-1000 µL) and for multiple dosing operations with an Eppendorf Multipette® E3 (0.1-50 mL). Constant larger quantities (>30 mL for water and batch solutions) were dosed with bottle neck dispensers (5-50 mL VWR brand and 10-100 mL Dispensette® S). Solid reagents were weighed with a Sartorius analytical balance with 0.1 mg deviation. Reactions are performed in cylindrical 110 mL Xpress Plus PTFE tubes belonging to CEM MARS6 microwave reactor setup.

Separation steps were performed with 45 mL PP-centrifugation tubes for each 10 min at 7830 rpm (G-force: 7200) soft ramp in an Eppendorf centrifuge 5430 with a fixed angle rotor (F-35-6-30; 45°).

Reaction vessels were cleaned first mechanically with water then immersed in KOH / *iso*propanol and nitric acid bath consecutively. After washing with deionized water and Millipore water reactors were dried prior to use.

### Analytics

Standard elemental analysis was carried out to determine the elemental composition. Composition of constitutes is calculated with the solver function implemented in Microsoft Excel. Microscopic images of the particles were taken via scanning electron microscopy with a Hitachi TM-1000 tabletop microscope with 15 keV accelerating voltage.

Particle size and size distributions of the rhenium (VII) sulfide particles were determined via dynamic light scattering (DLS) using a dispersion of the particles in water with a temperature of 25°C as a sample. The measurement was carried out with a Malvern Zetasizer Nano. Rhenium sulfide particle samples were dispersed in water and ultrasonicated directly prior to the measurement.

All measurements were performed with the following settings:
material: default refractive index (RI) 0.1, absorption (Abs) 0.1; dispersion in water, temperature 25.0°C; 60s temperature equilibration time; ZEN0040 sample cell; 173° backscatter detection; Run duration 10s; run number: 11per measurement, number of measurements: 3; Positioning method: Seek for optimum position; automatic beam attenuation; laser wavelength 633 nm.

Given size values are mean values of these three measurements a eleven runs a ten seconds. Due to particle agglomerations during the measurements bimodal size distributions could occur exhibiting a second size fraction peaking around 2-3 µm. As SEM measurements confirm, this DLS specific observation is a measurement artefact detecting oligomeric aggregates of primary particles. Therefore, these bimodal curves are also within specifications.

### Reagents

All reactions and washing procedures were performed with ultrapure Millipore water (R=18.2 MΩcm) referred to hereafter as "water". Metallic rhenium powder was purchased from Sigma-Aldrich (99.995% trace metal basis; Product No: 204188) or Alfa Aesar (-325 mesh, 99.99% trace metal basis, Product No: 10313)Thioacetamide (TAA) was obtained from Alfa Aesar (Product No: A12926, 98%) and from Sigma-Aldrich (Product No: 163678 99%). Poly(vinylpyrrolidone) (PVP) was obtained from Sigma Aldrich (product No: 856568, -55,000 M_{W}) and used as a 2.55 mM aqueous solution. Nitric acid (65%, Suprapur® for trace analysis, Supelco®) was purchased from Merck Millipore. The amount of used nitric acid was dosed volumetrically with a Multipette E3.

Ammonium perrhenate was synthesized according to literature procedures as follows:

NH₃ + HReO₄ → NH₄ReO₄

Aqueous HReO4 is allowed to react with the free base until change of color of methyl red, during the reaction crystals may already precipitate. After evaporation of the solvent, precipitated crystals are sucked off, washed with small amounts of ice-cooled water and dried at 110°C. The salt can be recrystallized from water.

Elemental analysis of the used reagents was performed to determine purity and composition of TAA and PVP. The results are shown in table 1.

**Table 1: Composition of used reagents calculated on elemental analysis data.**

| Reagent | Purity | Impurity (amount) |
|---|---|---|
| TAA | 99.1 % | Water (0.9%)¹ |
| PVP | 88.3% | Water (11.7%) |

| | | |
|---|---|---|
| ¹Trace amounts of insoluble elemental sulfur could be present. | | |

### Particle work-up after synthesis

Irrespective of the synthetic procedure (A, B or C), the obtained particles were transferred into 45 mL centrifugation tubes and successively exposed to at least five purification cycles consisting of centrifugation, decantation and redispersion in ultrapure water. In the last cycle, the supernatant was removed after centrifugation and resulting particles are dried at elevated temperatures and respective analyses were performed.

### 2. Large-scale microwave-assisted preparation (method A)

### General Information

Large-scale microwave-assisted syntheses were performed using a CEM MARS6 module for focused synthesis with implemented stirring, with the temperature of each vessel being constantly monitored by IR sensors. All reaction tubes were placed in a 24 slots turntable each mantled with Kevlar-based security sleeves. Reactions were performed using cross shaped magnetic stirring bars (8 x 20 mm). Table 2 shows the applied instrument and program settings of the microwave reactor.

**Table 2: Applied settings for microwave reactor CEM MARS6**

| Microwave reactor settings for full production capacity | |
|---|---|
| Program | Standard |
| Content | Organic |
| MW power | 1800 W |
| Target temperature | 125 °C |
| Safe guard | 200 °C |
| Ramp time | 25 min |
| Stirring | High (800 rpm) |
| Average time for heat up | 19:00 - 20:00 min |
| Hold time at target T | 15 min |
| Cool down threshold | 80 °C |

### Preparation of batch solution B1

PVP and TAA were dosed in a batch solution in the following called "B1". For the preparation of 500 mL of B1, 13.89 g TAA was dissolved in 450 mL water. To free the resulting solution from potential sulfur impurities, it was filtered through a folded filter paper. To the filtrate, 11.1 mL PVP solution (2.55 mM) was added and the volume of the combined solution was adjusted to 500 mL. The prepared batch solution B1 can be stored in closed flasks for several weeks minimum.

### Synthetic Procedure for maximum scale (1.15 q rhenium)

### Rhenium dissolution

The entire Re dissolution was conducted in a fume hood including personal safety equipment. 1.152 g rhenium powder were placed in a 60 mL narrow shape beaker together with a stirring bar, 12 mL water was added. With a magnetic stirring plate, to the black suspension 15.6 mL nitric acid was added in three equal portions with approximately 1 min between each dosage. The last dosage was added after the black suspension had turned into a clear green solution. 10 min after complete Re dissolution, the volume of the resulting yellowish solution was adjusted to 30 mL and the dilution step (see the following subchapter) is performed.

### MW-assisted Rhenium sulfide production

1.3 mL of the acidic rhenium solution was transferred to each of the MW reaction tubes together with a stirring bar. First, to all tubes 34 mL water, then 35 mL batch solution B1 were added. All solutions were stirred for at least 30 s after each dosage. Reaction tubes were carefully closed with PTFE lids and sealed with screw caps using a torque wrench. All reaction tubes were consequently placed into the turntable according to the operationsinstructions of the used instrument.

The reaction was performed with the described instrument settings. Generally, the heating phase requires -20 min to reach 125 °C, followed by 15min hold time at this temperature. After cool-down, the reaction mixtures were transferred into centrifugation tubes and exposed to the typical purification procedure described above to obtain rhenium sulfide nanoparticles with 0.5-1.5 µm average particle size, narrow size distribution (see SEM images and graphs in the Figures) and high reaction yields (80-95%).

### Flexible production scale

To lower Re₂S₇ production per batch, it is possible to reduce the number of used reaction tubes. To obtain equal heating rates, left out reaction tubes can be replaced by dummy tubes with equal filling (70 mL) containing aqueous sodium chloride solutions (6.66 g L⁻¹) mimicking similar MW absorption properties.

Shorter ramp times can be achieved by using less reaction tubes (minimum 8 tubes) affording similar yields and particle size distributions. According to the user instructions, the Kevlar safety sleeves were removed for all unused slots at the turntable to avoid errors intemperature sensing. For MW runs including less than 24 tubes, the loading was performed according to CEMs operational manual.

### Analysis of product from large-scale preparation

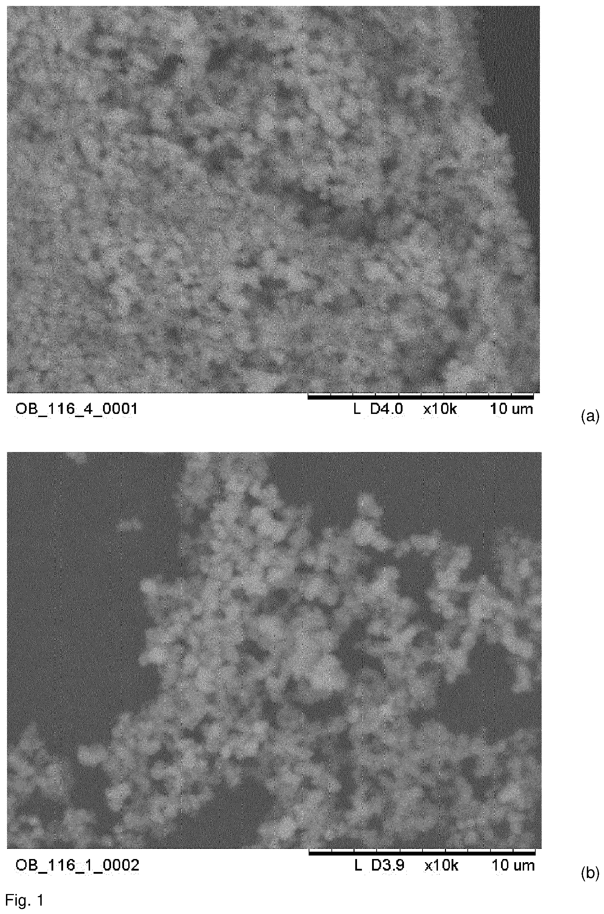

Fig.1a and b show scanning electron microscopy (SEM) images of rhenium sulfide particles from the MW-assisted synthesis described above.

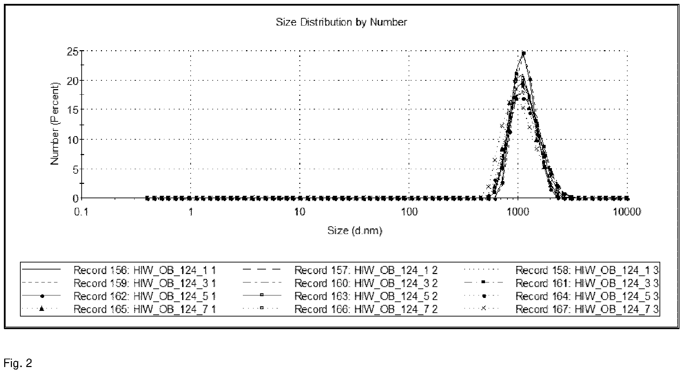

Further, Fig. 2 shows the size distribution by number of the rhenium sulfide particles determined by dynamic light scattering, indicating a narrow particle size distribution and average particle size of about 1.0 µm (standard deviation of ±0.4 µm). Three measurements of the same samples and four measurements of different samples are displayed indicating particle homogeneity.

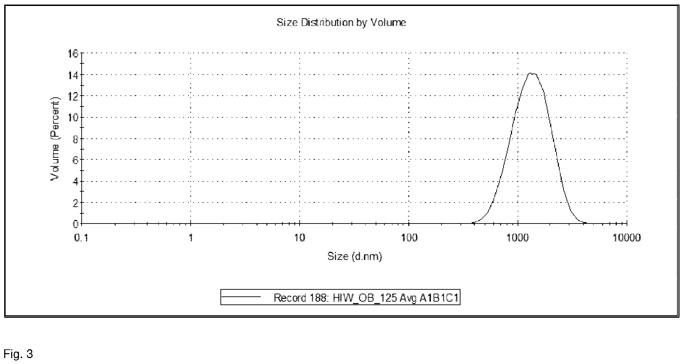

Fig. 3 shows an exemplary volume-based particle size distribution of a whole Re₂S₇ production using enriched rhenium-187.

In accordance with the above described method rhenium sulfide nanoparticles are obtained with 0.5 to 1.5 µm average particle size and a narrow distribution. Further, high reaction yields of 80 to 95% are achieved.

### 3. Small-scale microwave-assisted preparation (method B)

### General Information

Microwave-assisted syntheses are performed using a CEM Discover module for focused synthesis with implemented stirring, constant temperature monitoring by an IR sensor and "Power Max" function (pressurized gas stream is cooling reaction vessel during reaction). Reactions are performed in 65 mL flat-bottomed glass reactors (ACE-glass®, cylindric, inner diameter 2.8 cm, height 11 cm, wall thickness 4 mm, front sealed Teflon plug).
Reactions are performed using cylindrical magnetic stirring bars (I = 20 mm, ø = 8 mm). Table 3 shows the applied instrument and program settings of the microwave reactor.

**Table 3: Applied settings for microwave reactor CEM discover.**

| Microwave reactor settings | |
|---|---|
| Program | Standard, open vessel |
| Max. MW power | 300 W |
| Max. temperature | 100 °C |
| Power max function¹ | Enabled |
| Stirring | High (800 rpm) |
| Average time for heat up | 2:40 min ±0:10 |
| Reaction time at 100 °C | 15 min |
| Cool down threshold | 50 °C |

| | |
|---|---|
| ¹External air stream is cooling vessel during reaction | |

### Synthetic Procedure for small-scale syntheses (31 mg rhenium)

45 mg ammonium perrhenate and 625 mg thioacetamide were weighed in and dissolved in 44 mL Millipore water. The solution was filtered through a fold filter paper to remove trace amounts of elemental sulfur stemming from TAA. To the solution, 250 µL PVP solution was dosed directly into the liquid phase and the resulting mixture was stirred until a clear solution had formed. After addition of 620 µL nitric acid (3.95M), the reaction vessel was closed tightly and placed in the microwave reactor. The reaction was performed with the described instrument settings. Generally, the heating phase requires -2:40min until 100°C are reached, followed by 15min reaction time at this temperature. The reaction vessel was cooled to ambient temperature *via* gas stream or by water cooling, alternatively. The reaction mixture was transferred into a centrifugation tube and purified according to the typical procedure described above.

### Analysis of product obtained from small-scale preparation

As can be seen, the obtained rhenium sulfide nanoparticles have a narrow particle size distribution and an average particle size of 1.7 µm with a standard deviation of ±0.3 µm.

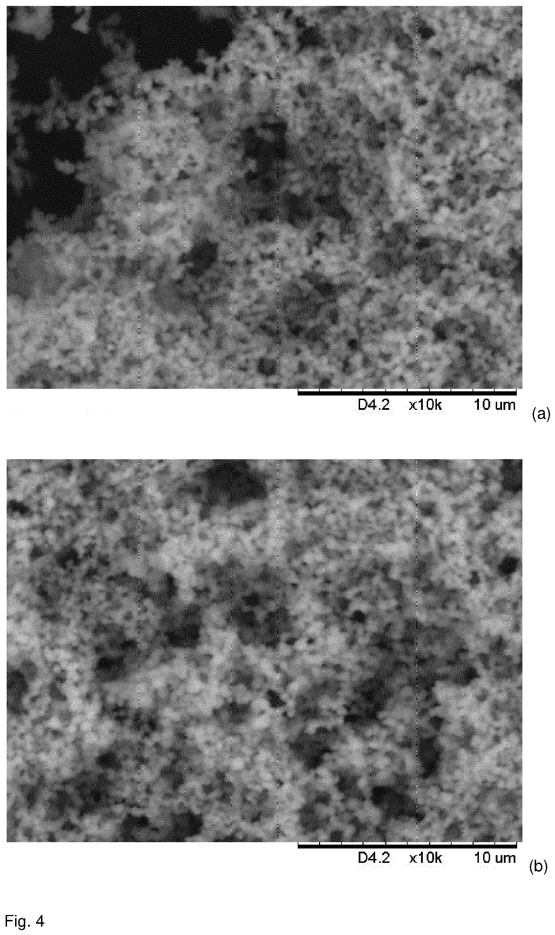

Fig. 4a and b show scanning electron microscopy (SEM) images of rhenium sulfide particles obtained from small-scale MW-assisted synthesis.

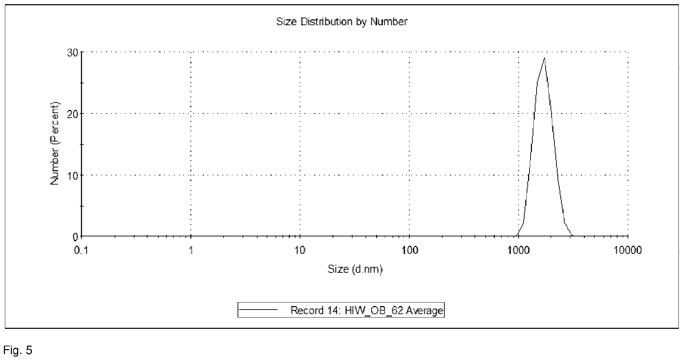

Fig. 5 shows the size distribution by number of the rhenium sulfide particles determined by DLS as described above, the curve indicates a narrow particle size distribution and an average particle size of 1.7 µm (standard deviation of ±0.3 µm). The curve represents the average of three individual samplings of each three measurements.

### 4. Conventionally heated reference preparation (method C)

### General Information

Reactions were performed in 65 mL flat-bottomed glass reactors (ACE-glass®, cylindric, inner diameter 2.8 cm, height 11 cm, wall thickness 4 mm, front sealed Teflon plug). For heating, the reaction vessel was placed into a preheated oil bath for the indicated time.

### Synthetic Procedure for conventionally heated preparation (10.4 mg rhenium)

15 mg ammonium perrhenate and 625 mg thioacetamide were weighed in and placed, together with a stirring bar, in the glass reactor. 45 mL Millipore water were added. 25 µL PVP solution were dosed directly into the liquid phase and the resulting mixture was stirred until a clear solution had formed. After addition of 210 µL nitric acid, the reaction vessel was closed tightly and placed in the preheated oil bath at 110 °C for 60 min (±2 min). Afterwards, the glass reactor was cooled down to ambient temperature with a water bath. The reaction mixture was transferred into a centrifugation tubes and purified accordingly.

### Analysis of product obtained from conventional preparation

The obtained rhenium sulfide particles from conventionally heated preparation possess mainly 1 to 4 µm particle size and a much broader particle size distribution with the presence of a bimodal distribution: The coexistence of larger (-2-4 µm) and smaller (<0.2 µm) particles.

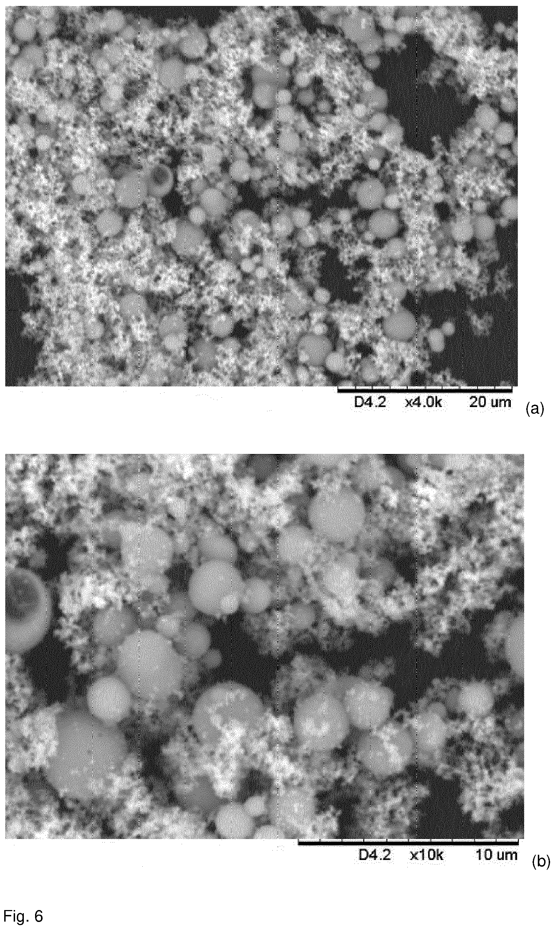

Fig. 6a and b show scanning electron microscopy (SEM) images of rhenium sulfide particles prepared by conventional heating. The obtained particles have heterogeneously distributed particle sizes.

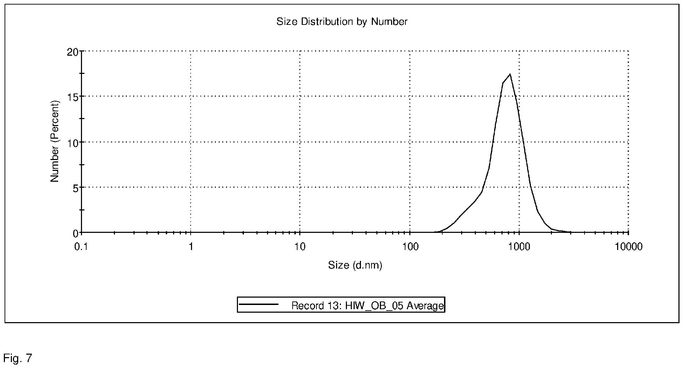

Fig. 7 shows the number-based particle size distribution of the rhenium sulfide particles prepared by conventional heating, the broad particle size distribution with small and large particles indicates heterogeneous particle sizes.

### 5. Comparison of conventional and microwave-assisted preparations

A direct comparison of the conventionally heated preparation (reference preparation) and the microwave-assisted preparation was conducted utilizing identical reagent concentrations. Used amounts and obtained results are listed in the following. SEM images were recorded with identical magnification. Differences in image brightness and contrast may arise due to automatic adjustment and are without scientific meaning.

### Synthetic Procedure for comparison of preparations (10.4 mg rhenium)

Preparations were conducted according to previous descriptions utilizing the following reagent amounts: 56 pmol ammonium perrhenate, 31.5 µmol PVP in form of 25 µL solution, 8.3 mmol thioacetamide, 3 mmol nitric acid (3.95 M), 45 mL Millipore water.

Samples were heated to 110°C for 1 h (conventional heating), 11 min MW-heating with use of identical vessel, stirring bar and stirrer speed. Other experiments have shown that longer reaction times in MW-assisted heating do not influence the particle size or its distribution.

### Analysis from conventional preparation

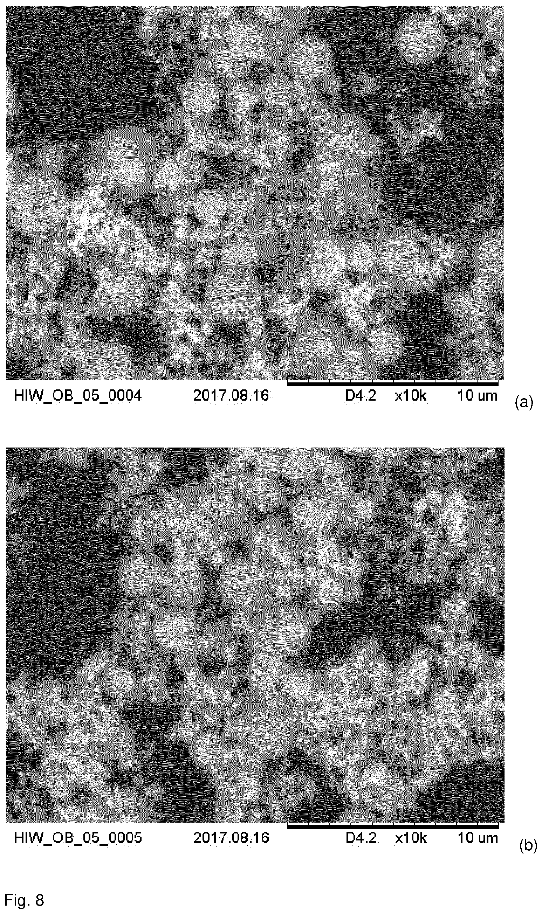

Fig. 8a and b show scanning electron microscopy (SEM) images of rhenium sulfide particles produced by conventional heating. Images reveal a broad particle size distribution. Particles of up to about 4 to 5 µm are embedded in relatively fine "sleet".

### Analysis of obtained particles produced from microwave-assisted preparation

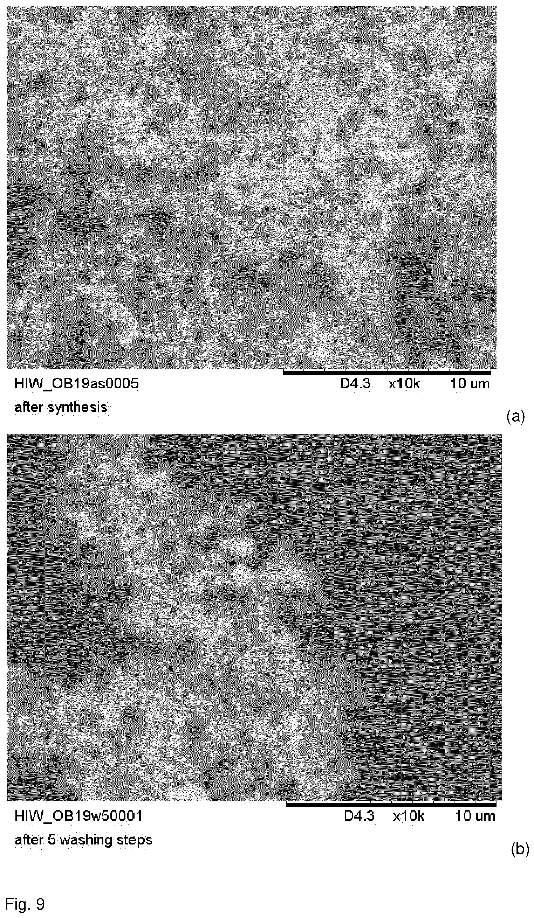

Fig. 9a and b show scanning electron microscopy (SEM) images of rhenium sulfide particles produced by MW-assisted heating. Images reveal uniform, homogeneous particles without the formation of isolated "big" particles or very fine sleet.

### Investigation of possible influence of temperature

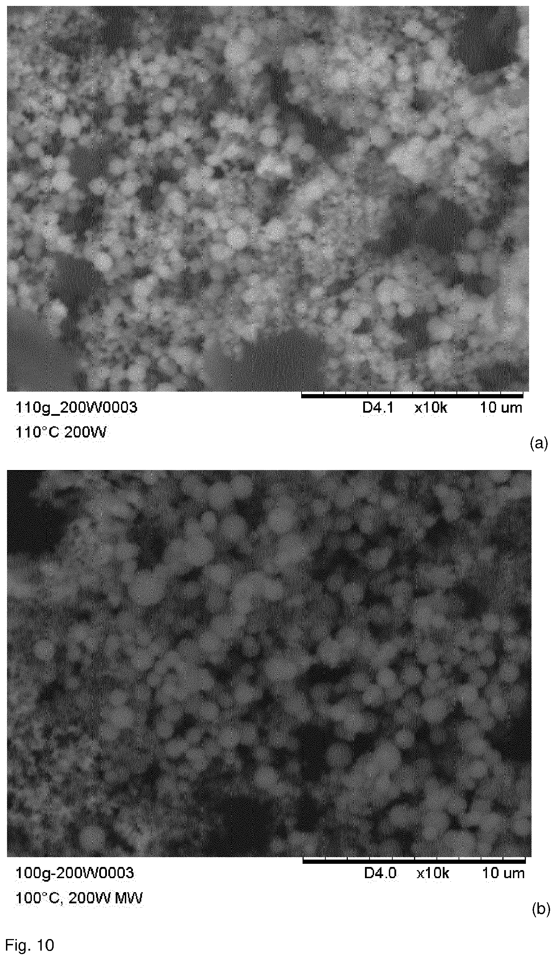

In small scale reactions utilizing different reagent ratios, the individual temperature influence was further investigated. Fig. 10 shows a comparison of small-scale MW-assisted synthesis at 110°C (Fig. 10a) and 100°C (Fig. 10b). Both temperatures provide particles with identical particle size and distribution.

Moreover, a comparison of small- and large-scale preparations utilizing microwave heating reveals the effect of the temperature ramp: As heating rates in small-scale reactions are high, formation of larger particles can be observed (Figure 10). With lower heating rates of large-scale reactions, mainly smaller particle sizes are observed (Figure 9). As appreciated by the skilled reader, heating rates correlate with the ratio of the available power input and the weight loading. Therefore, lower heating rates are typically obtained in large scale productions if the power input is kept at the same level.

### 6. Preparations using enriched Re-187

The following table 4 shows the intensity, number and volume-based particle size distributions for a sample of Re-187 enriched rhenium sulfide, averaged on multiple measurements produced by a large-scale method in analogy to method A described above.

**Table 4: Particle size distributions for a sample of enriched rhenium sulfide averaged on multiple measurements produced by large-scale method.**

| **Size distribution by:** | **Mean particle size [µm]** | **Standard deviation [µm]** |
|---|---|---|
| Intensity | 1.26 | ±0.48 |
| Number | 0.95 | ±0.37 |
| Volume | 1.42 | ±0.55 |

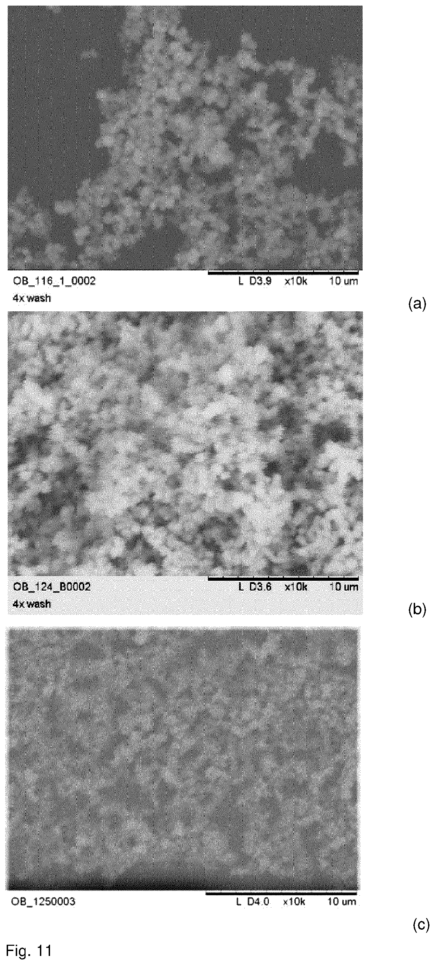

Figure 11a and 11b show SEM images of large-scale MW-assisted rhenium sulfide particles obtained from different productions with 24 tubes loading reveal the highly reproducible production process. Fig. 11c shows a sample of enriched rhenium-187 sulfide.

## Claims

1. A process for the preparation of particles comprising rhenium (VII) sulfide, which process comprises
providing a reaction solution comprising perrhenic acid or a salt thereof, a Bronsted acid other than perrhenic acid and a sulfide source, and
heating the reaction solution comprising perrhenic acid or a salt thereof, a Bronsted acid other than perrhenic acid, and a sulfide source by exposure to microwave radiation to convert the perrhenic acid or the salt thereof to particles comprising rhenium (VII) sulfide.

2. The process according to claim 1, wherein the sulfide source is thioacetamide.

3. The process according claim 1 or 2, wherein the reaction solution comprising perrhenic acid or a salt thereof, a Bronsted acid other than perrhenic acid and a sulfide source further comprises a capping agent.

4. The process according to claim 3, wherein the capping agent is polyvinylpyrrolidone.

5. The process according to any of claims 1 to 4, wherein the reaction solution comprises nitric acid as the Bronsted acid other than perrhenic acid.

6. The process according to any of claims 1 to 5, wherein the reaction solution comprises nitric acid as the Bronsted acid other than perrhenic acid, and wherein providing the reaction solution comprising perrhenic acid or a salt thereof, nitric acid, a sulfide source and optionally a capping agent comprises the steps of:
(i) providing a solution comprising perrhenic acid and nitric acid by dissolving and oxidizing metallic rhenium in a solution comprising nitric acid, and
(ii) combining the sulfide source and optionally the capping agent with the perrhenic acid and the nitric acid comprised in the solution provided in step (i) to provide a reaction solution comprising perrhenic acid, nitric acid, a sulfide source and optionally a capping agent.

7. The process according to claim 6, wherein the sulfide source and optionally the capping agent are combined with the perrhenic acid and the nitric acid comprised in the solution provided in step (i) without prior isolation of the perrhenic acid obtained by dissolving and oxidizing the metallic rhenium or of a salt thereof.

8. The process according to any of claims 1 to 7, wherein the reaction solution comprising perrhenic acid or a salt thereof, a Bronsted acid other than perrhenic acid, a sulfide source and optionally a capping agent is heated by the exposure to microwave radiation to a reaction temperature of 60°C to 150°C.

9. The process according to claim 8, wherein the time span between the start of the exposure to microwave radiation and the time when the temperature of the reaction solution reaches the reaction temperature is in the range of 0.1 to 30 minutes.

10. The process according to any of claims 1 to 9, wherein the obtained rhenium (VII) sulfide particles have a particle size distribution with a polydispersity D in the range of 1.0 to 2.5.

11. A process for the preparation of a composition comprising a plurality of particles comprising rhenium (VII) sulfide in a carrier material, said process comprising preparing particles comprising rhenium (VII) sulfide in accordance with the process of any of claims 1 to 10, and incorporating the obtained rhenium (VII) sulfide particles into a carrier material.

12. A composition comprising a plurality of particles comprising rhenium (VII) sulfide, wherein the particles comprising rhenium (VII) sulfide have a volume average particle size, of 0.05 to 5 µm, and wherein the particles comprising rhenium (VII) sulfide have a unimodal particle size distribution and a polydispersity Ð in the range of 1.0 to 2.5.

13. The composition according to claim 12, wherein the particles comprising rhenium (VII) sulfide comprise rhenium-186 or rhenium-188.

14. The composition according to claim 13 for use in medicine.

15. The composition according to claim 13 for use in the treatment of non-melanoma skin cancer selected from basal cell carcinoma and squamous cell carcinoma.
